# EUROPEAN PATENT SPECIFICATION

(11) **EP 2 887 924 B1**
(45) Date of publication and mention of the grant of the patent: **29.03.2017**
(21) Application number: 13770967.1
(22) Date of filing: 27.08.2013
(51) Int. Cl.: A61K 9/20, A61K 31/444

(54) **PHARMACEUTICAL COMPOSITIONS OF ETORICOXIB**
PHARMAZEUTISCHE ZUSAMMENSETZUNGEN VON ETORICOXIB
COMPOSITIONS PHARMACEUTIQUES D'ÉTORICOXIB

(30) Priority: 27.08.2012 IN MM24772012
(43) Date of publication of application: 01.07.2015
(73) Proprietor: Cadila Healthcare Limited, Ahmedabad 380 015, Gujarat (IN)
(72) Inventor: BHUSHAN, Roy Sunilendu, Ahmedabad Gujarat 380 015 (IN); KRISHNAJI, Kulkarni Sushrut, Ahmedabad Gujarat 380 015 (IN); RAJNIKANT, Mehta Pavak, Ahmedabad Gujarat 380 015 (IN); RITESH, Kapoor, Ahmedabad Gujarat 380 015 (IN); BHARAT, Maheshwari Rajeshkumar, Ahmedabad Gujarat 380 015 (IN)
(74) Representative: Hamer, Christopher K.
(86) International application number: PCT/IB2013/001850
(87) International publication number: WO 2014/033526

(56) References cited:
- WO-A1-2012/163839
- WO-A2-2006/052503
- WO-A2-2010/101485
- SHAOJUN SHI ET AL: "Clinical use and pharmacological properties of selective COX-2 inhibitors", EUROPEAN JOURNAL OF CLINICAL PHARMACOLOGY, SPRINGER, BERLIN, DE, vol. 64, no. 3, 13 November 2007 (2007-11-13), pages 233-252, XP019585530, ISSN: 1432-1041

## Description

### FIELD OF THE INVENTION

The present invention relates to pharmaceutical composition of crystalline Form I of etoricoxib for oral administration. More particularly, it relates to a pharmaceutical composition of crystalline Form I of etoricoxib, which comprises dry granulated particles of crystalline Form I of etoricoxib.

### BACKGROUND OF THE INVENTION

Etoricoxib is a selective COX-2 inhibitor which has been shown to be as effective as nonselective non-steroidal anti-inflammatory drugs in the management of chronic pain in rheumatoid arthritis, osteoarthritis and other COX-2 mediated disorders. Etoricoxib is 5-chloro-6'-methyl-3-[4-methylsulfonyl)phenyl]-2,3'-bipyridine having structural Formula I.

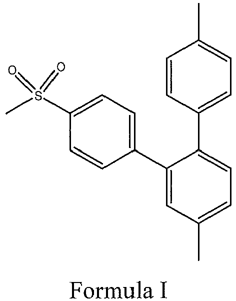

Etoricoxib is a potent and selective cyclooxygenase-2 (COX-2) inhibitor. Etoricoxib belongs to a class of drugs known as COX-2 inhibitors that are used in the treatment of COX-2 mediated disorders. The therapeutic application of etoricoxib as a COX-2 inhibitor is disclosed in WO 96/10012 and WO 96/16934. This compound is disclosed in US Patent No. 5,861,419.

A process for preparation of this compound is disclosed in US Patent No. 6,040,319.

PCT publication No. WO 01/992230 discloses Form V of etoricoxib. It further discloses five polymorphic forms, one amorphous form and two hydrated forms. Thus it describes eight new forms of etoricoxib.

PCT publication No. WO 2005/085199 discloses etoricoxib Form IX, Form X, Form XI, Form XII, Form XIII, Form XIV, Form XV and Form XVI.

PCT publication No. WO 2006/043025 discloses granular compositions comprising solidified melt granules of COX-2 selective inhibitor.

PCT publication No. WO 2006/052503 discloses a wurster granulation process, a process for granulating particles by subjecting the particles to a repeated circulating movement in which particles are subjected to a spray of droplets of granulation solution.

Compositions of etoricoxib may be prepared by techniques known in the art i.e. wet granulation, dry granulation, direct compression or melt granulation. We have found that when the composition of etoricoxib is prepared by wet granulation using water, there exists a polymorphic conversion of etoricoxib in the composition, which may lead to decrease in solubility and/or stability of the final composition. We have surprisingly found that there is no polymorphic conversion of etoricoxib in the composition while the composition is prepared by dry granulation process and thus the composition remains stable and the solubility of etoricoxib is not hampered.

### BRIEF DESCRIPTION OF DRAWINGS

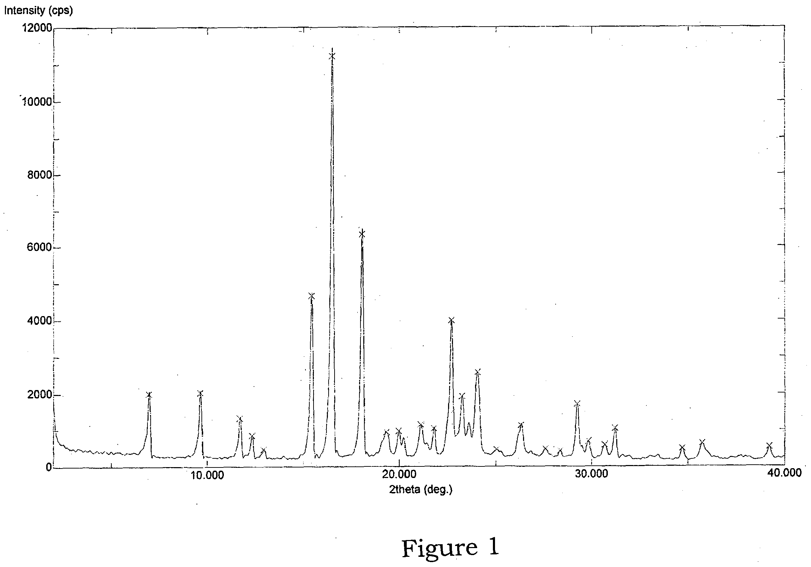

**Figure 1** illustrates an XPRD pattern of crystalline etoricoxib active ingredient.

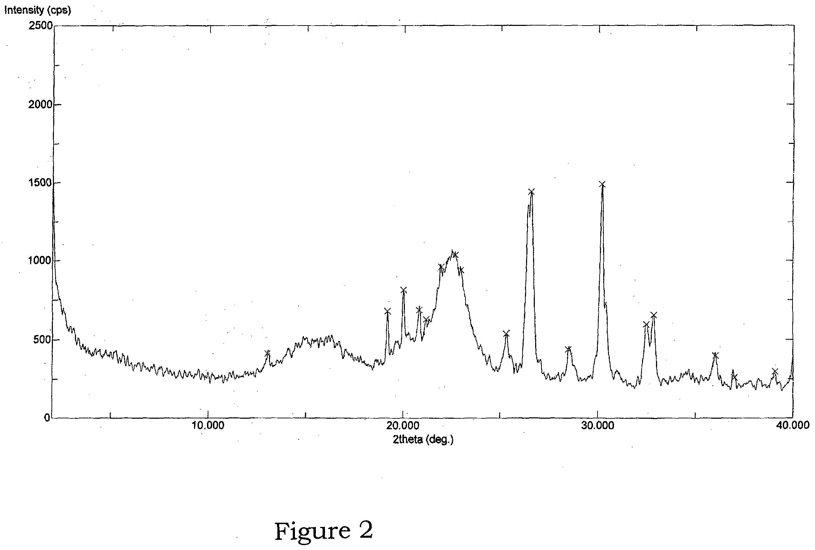

**Figure 2** illustrates an XPRD pattern of placebo.

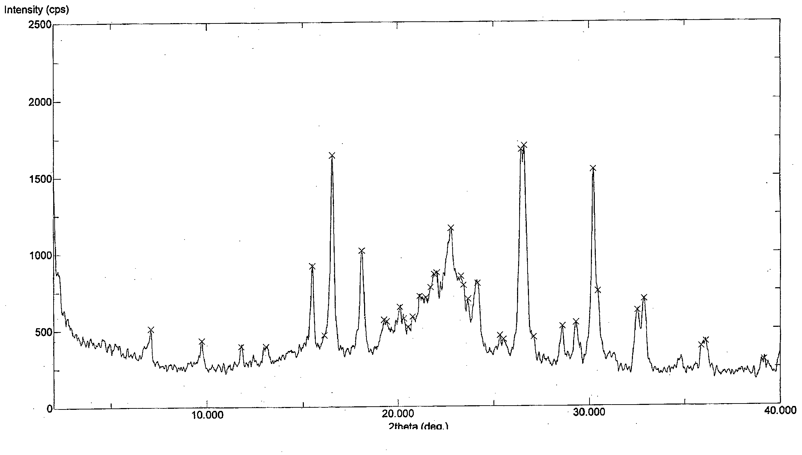

**Figure 3** illustrates an XPRD pattern of tablet containing crystalline etoricoxib active ingredient.

### SUMMARY OF THE INVENTION

In one general aspect, there is provided a pharmaceutical composition comprising crystalline Form I of etoricoxib, at least one solubility enhancing agent which is a surfactant selected from the group consisting of anionic surfactants, cationic surfactants, non-ionic surfactants and zwitterionic surfactants or mixtures thereof, and one or more pharmaceutically acceptable excipients,
wherein the anionic surfactant is selected from the group consisting of salts of aliphatic monoesters of sulfuric acid and soaps; sulfonated aromatic agents and salts thereof; alkyl naphthalene sulfonates; sulphosuccinates; sulfated polyoxyethylated alcohols; sulfated oils;
dioctyl sodium sulfosuccinate; phosphatidyl choline, phosphatidyl glycerol, phosphatidyl inosine, phosphatidylserine, phosphatidic acid and their salts; glyceryl esters; and bile acids and salts thereof;
wherein the non-ionic surfactant is selected from the group consisting of polyoxyethylene fatty alcohol ethers; polyoxyethylene sorbitan fatty acid esters; polyoxyethylene fatty acid esters; sorbitan esters; glycerol monostearate; polyethylene glycols; polypropylene glycols; cetyl alcohol; cetostearyl alcohol; stearyl alcohol; aryl alkyl polyether alcohols; polyoxyethylene-polyoxypropylene copolymers; and polaxamines; and
wherein the composition is prepared by dry granulation and said etoricoxib retains its original polymorphic form.

In another general aspect, there is provided a process for preparing a pharmaceutical composition comprising:
a. mixing crystalline Form I of etoricoxib, a solubility enhancing agent which is a surfactant selected from the group consisting of anionic surfactants, cationic surfactants, non-ionic surfactants and zwitterionic surfactants or mixtures thereof, and one or more pharmaceutical excipients;
   wherein the anionic surfactant is selected from the group consisting of salts of aliphatic monoesters of sulfuric acid and soaps; sulfonated aromatic agents and salts thereof; alkyl naphthalene sulfonates; sulphosuccinates; sulfated polyoxyethylated alcohols; sulfated oils; dioctyl sodium sulfosuccinate; phosphatidyl choline, phosphatidyl glycerol, phosphatidyl inosine, phosphatidylserine, phosphatidic acid and their salts; glyceryl esters; and bile acids and salts thereof;
   wherein the non-ionic surfactant is selected from the group consisting of polyoxyethylene fatty alcohol ethers; polyoxyethylene sorbitan fatty acid esters; polyoxyethylene fatty acid esters; sorbitan esters; glycerol monostearate; polyethylene glycols; polypropylene glycols; cetyl alcohol; cetostearyl alcohol; stearyl alcohol; aryl alkyl polyether alcohols; polyoxyethylene-polyoxypropylene copolymers; and polaxamines;
b. dry granulating the above mixture to form desired granules; and
c. lubricating the granules of step (b) with a lubricant and converting the resulting mixture into a solid dosage form.

Embodiments of the pharmaceutical composition may include one or more of the following features. For example, the pharmaceutical composition may include one or more pharmaceutically acceptable excipients selected from binders, fillers, lubricants, disintegrants, glidants, antioxidants, solvents, flavors, sweeteners and the like.

The details of one or more embodiments of the invention are set forth in the description below. Other features, objects and advantages of the invention will be apparent from the description.

### DETAILED DESCRIPTION OF THE INVENTION

The inventors of the present invention have surprisingly found that the stable composition of etoricoxib can be prepared by dry granulation process to avoid polymorphic conversion of etoricoxib in the composition.

The term "dry granulation" means the process of blending bulk etoricoxib with at least one excipient. The blend is then compressed, or compacted to form a compressed material or "compact". This material is then broken apart to form granules by crushing, grinding or cutting into dry granulated particles. Optionally, the particles may be further processed. Crushing, grinding, or cutting processes involve an operation that reduces the size of the compressed material such as accomplished by milling or by other operations known to those skilled in the art.

A "compact" is a compressed material formed by processing etoricoxib and optional excipients by slugging or by roller compaction.

"Granules" or "dry granulated particles" are defined herein as particles containing etoricoxib and one or more pharmaceutically acceptable excipients, that are formed by dry granulation process.

The pharmaceutical composition according to the invention comprises at least one solubility enhancer which is a surfactant. Further solubility enhancers are hydrocolloids such as cellulose derivatives (e.g. hydroxypropyl methyl cellulose, hydroxypropyl cellulose, hydroxyl methyl cellulose); polymers such as N-vinyl-2-pyrrolidone, polyvinyl pyrrolidone; copolymers such as copolymer of vinylpyrrolidone (VP) and vinylacetate (VA). Preferably, solubility enhancing agent used in the pharmaceutical composition of etoricoxib is sodium lauryl sulphate.

The "surfactants" which are used for preparing pharmaceutical composition of crystalline Form I of etoricoxib according to the invention are selected from the group consisting of anionic, cationic, non-ionic or zwitterionic surfactants or mixtures thereof, wherein the anionic surfactant is selected from the group consisting of salts of aliphatic monoesters of sulfuric acid and soaps; sulfonated aromatic agents and salts thereof; alkyl naphthalene sulfonates; sulphosuccinates; sulfated polyoxyethylated alcohols; sulfated oils; dioctyl sodium sulfosuccinate; phosphatidyl choline, phosphatidyl glycerol, phosphatidyl inosine, phosphatidylserine, phosphatidic acid and their salts; glyceryl esters; and bile acids and salts thereof; and the non-ionic surfactant is selected from the group consisting of polyoxyethylene fatty alcohol ethers; polyoxyethylene sorbitan fatty acid esters; polyoxyethylene fatty acid esters; sorbitan esters; glycerol monostearate; polyethylene glycols; polypropylene glycols; cetyl alcohol; cetostearyl alcohol; stearyl alcohol; aryl alkyl polyether alcohols; polyoxyethylene-polyoxypropylene copolymers; and polaxamines.

Suitable cationic surfactants may include one or more of quaternary ammonium compounds, such as benzalkonium chloride, cetyl trirnethyl ammonium bromide and dodecyl dimethyl ammonium bromide, hexadecyl (cetyl) trimethylammonium bromide, dodecyl pyridinium chloride, lauryl dimethyl benzyl ammonium chloride, acyl carnitine hydrochlorides, alkyl pyridinium halides, dodecylamine hydrochloride and the like.

Suitable anionic surfactants may include one or more of salts of aliphatic monoesters of sulfuric acid and soaps, such as potassium laurate; sodium dodecyl sulphate; alkyl polyoxyethylene sulfates; sodium alginates; sodium lauryl sulphate and sodium heptadecyl sulphate; sulfonated aromatic agents such as alkyl benzene sulfonic acids and salts thereof, such as tridecylbenzene sulphonic acid and the sodium and amino salts of dodecylbenzene sulphonic acid; alkyl naphthalene sulfonates, such as sodium butylnaphthalene sulphonate, sulphosuccinates such as sodium dioctyl sulphosuccinate and N-acyi-N-alkyl fatty acid taurates; sulfated polyoxyethylated
alcohols; sulfated oils; dioctyl sodium sulfosuccinate, phosphatidyl choline, phosphatidyl glycerol, phosphatidyl inosine, phosphatidylserine, phosphatidic acid and their salts, glyceryl esters, sodium carboxymethylcellulose, cholic acid and other bile acids (e.g., cholic acid, deoxycholic acid, glycocholic acid, taurocholic acid, glycodeoxycholic acid) or salts thereof, and the like. The preferred anionic surfactant for use in etoricoxib composition is sodium lauryl sulphate.

Suitable non-ionic surfactants may include one or more of polyoxyethylene fatty alcohol ethers (Macrogol and Brij), polyoxyethylene sorbitan fatty acid esters (Polysorbates), polyoxyethylene fatty acid esters (Myrj), sorbitan esters (Span), glycerol monostearate, polyethylene glycols, polypropylene glycols, cetyl alcohol, cetostearyl alcohol, stearyl alcohol, aryl alkyl polyether alcohols, polyoxyethylene-polyoxypropylene copolymers (poloxomers), polaxamines, methylcellulose, hydroxycellulose, hydroxy propylcellulose, hydroxypropylmethylcellulose, noncrystalline cellulose, polyvinyl alcohol, polyvinylpyrrolidone, and the like.

Suitable zwitterionic surfactants may include one or more of alkyl betaines, alkyl amidopropyl betaines, alkyl sulphobetaines, alkyl glycinates, alkyl carboxyglycinates, alkyl amphopropionates, alkyl amidopropyl hydroxysultaines, acyl taurates and acyl glutamates wherein the alkyl and acyl groups have from 8 to 18 carbon atoms such as cocamidopropyl betaine, sodium cocoamphoacetate, cocamidopropyl hydroxysultaine, sodium cocamphopropionate, and the like.

In a preferred embodiment of the invention, the nonionic surfactant is a polyoxyethylene and polyoxypropylene copolymer and preferably a block copolymer of propylene glycol and ethylene glycol. Such polymers are sold under the tradename Poloxamer also sometimes referred to as Pluronic. Among polyoxyethylene fatty acid esters is included those having short alkyl chains. For example, such a surfactant is selected from Solutol^{®}, HS 15, polyethylene-660-hydroxystearate or the like.

In an embodiment the particle size distribution of crystalline Form I of etoricoxib is such that D₉₀ is less than about 250 µm, D₅₀ is less than about 100 µm and D₁₀ is less than about 50 µm or any combination thereof.

The composition may be seal coated composition. Preferably, the composition is seal coated and finally film coated. The composition can be coated with ready color mix systems (such as opadry color mix systems).

Pharmaceutically acceptable excipients for use in the pharmaceutical composition comprise one or more diluents, bulking agents, binders, disintegrants, glidants, lubricants, sweeteners/taste masking agents, compression aids, colorants and flavors.

Suitable diluents or bulking agents which includes, but are not limited to, saccharides, including monosaccharides, disaccharides, polysaccharides and sugar alcohols such as arabinose, lactose, dextrose, sucrose, fructose, maltose, mannitol, erythritol, sorbitol, xylitol lactitol, and other bulking agents such as powdered cellulose, microcrystalline cellulose (e.g. MCC PH 101 and MCC PH 102), purified sugar and derivatives thereof. The formulation may incorporate one or more of the above bulking agents, preferably, lactose & microcrystalline cellulose forms the bulking agent.

Suitable binders or binders, which includes, but are not limited to, methyl cellulose, hydroxypropyl cellulose (HPC), hydroxypropyl methyl cellulose (HPMC), starch, gelatin, gum Arabic, ethyl cellulose, polyvinyl alcohol, tragacanth, sodium alginate and equivalents thereof.

Suitable disintegrants which includes, but are not limited to, croscarmellose sodium, crospovidone, sodium starch glycolate, com starch, potato starch, maize starch and modified starches, calcium silicates, low substituted hydroxy- propylcellulose.

Suitable lubricants and glidants which may include, but are not limited to, stearic acid and its derivatives or esters like sodium stearate, magnesium stearate and calcium stearate and the corresponding esters such as sodium stearyl fumarate; talc and colloidal silicon dioxide respectively.

Suitable taste masking agents may include one or more of polymers, sweeteners and flavors. Most preferred polymers include one or more of cellulose acetate, polymethacrylates, hydroxypropylmethyl cellulose, hydroxypropyl cellulose, hydroxylethyl cellulose; and the like. Most preferred sweeteners include but not limiting to one or more of aspartame, saccharin, sucralose, glycyrrhizin; and the like.

Suitable sweeteners that may be used, comprises saccharides such as sucrose, dextrose, glucose, maltose, dextrins, D-tagatose, trehalose, dried invert sugar, fructose, levulose, galactose, corn syrup solids, and the like, alone or in combination. Other examples of sweeteners comprise sodium saccharin; aspartame; sugarless sweeteners including polyhydric alcohols such as sorbitol, mannitol, xylitol, glycerol, hydrogenated starch hydrolysates, maltitol, isomaltitol, erythritol, lactitol and the like, alone or in combination.

Suitable flavors that may be used, comprise cinnamon, wintergreen, eucalyptus, spearmint, peppermint, menthol, anise as well as fruit flavors such as apple, pear, peach, strawberry, cherry, apricot, orange, watermelon, banana and the like; bean-derived flavors, such as coffee, cocoa and the like or mixtures thereof.

In one embodiment, etoricoxib composition may be prepared by granulating a blend of crystalline Form I of etoricoxib, a solubility enhancing agent as defined above and one or more pharmaceutical excipients. The resulting granules may be compressed to form tablets or filled in hard gelatin capsules.

To prepare the blend, the various components may be weighed, delumped. The mixing may be carried out for a sufficient period of time to produce a homogeneous blend. Lubricant may be added in one, or multiple steps, prior to and/or after initial blending of the etoricoxib or pharmaceutically acceptable salt thereof and other excipients. Afterwards, the final mixing may be carried out. The blend may be stored for later use.

The components of the blend, including the crystalline Form I of etoricoxib, and one or more pharmaceutically acceptable excipients, may be combined by blending, mixing, stirring, shaking, tumbling, rolling or by any other methods of combining the formulation components to achieve a homogeneous blend. It is preferable that the etoricoxib and excipients are combined under low shear conditions in a suitable apparatus, such as a V -blender, tote blender, double cone blender or any other apparatus capable of functioning under preferred low shear conditions. Lubricant is typically added in the last step.

The invention should not be considered limited to these particular conditions for combining the components and it will be understood, based on this disclosure that the advantageous properties can be achieved through other conditions provided the components retain their basic properties and substantial homogeneity of the blended formulation components of the formulation is otherwise .achieved without any significant segregation.

In another embodiment for preparing the blend, the components may be weighed and placed into a blending container. Blending may be performed for a period of time to produce a homogenous blend using suitable mixing equipment. Optionally, the blend may be passed through a mesh screen to delump the blend. The screened blend may be returned to the blending container
and blended for an additional period of time. Lubricant may then be added and the blend mixed for an additional period of time.

The blend, of the present invention, may be then compressed, or compacted, to form a compact. Prior to compression, the blend may be subjected to a precompression step such as on a rotary tablet press. Compression of the blend to form granules may be accomplished by techniques known in the art including slugging where the blend may be introduced into dies comprising one or more punch faces that are installed on a press such as a tablet press and pressure may be applied to the blend by the movement of one or more punch faces in the die. Dry granulation may also be performed through the use of a roller compactor. A roller compactor generally incorporates two or more rollers adjacent and parallel to each other with a fixed or adjustable gap between the rollers. A hopper or other feeding device deposits blend between the moving rollers which act to compact the blend into a compacted material. Roller compactors are typically equipped with dividers that cut or otherwise divide the compacted material emerging from the roller compactor into ribbons. An example of a roller compactor is TF-Mini 15 Roller Compactor (Vector Corporation, Marion, IA, Freund).

The compact may be then broken apart to form granules, typically by suitable mechanical means, such as by crushing, grinding or cutting.

Though generally not required with dry granulated tablets, in an alternate embodiment the dry granulated tablet may comprise an amount of glidant that is less than about 3% by weight, based on the tablet weight.

In a further embodiment, the direct compression tablet may comprise an amount of glidant that is less than about 1% by weight, based on the tablet weight. In an even further embodiment, the tablet may comprise an amount of glidant that is less than about 0.5% by weight, based on the weight of the glidant.

Suitable glidants include magnesium trisilicate, powdered cellulose, starch, talc, tribasic calcium phosphate, stearate salts and colloidal silicon dioxide. Most preferred glidants are talc, magnesium stearate and colloidal silicon dioxide.

If desired, the tablet may be coated. The reasons for coating a tablet may include masking the taste of the drug, making tablets easier to swallow, protection against chipping during packaging, a barrier for moisture or light to improve product stability, and enhancing product appearance or recognition.

The invention further provides a method of treating pain and inflammatory signs comprising administering to said subject a pharmaceutical composition of crystalline Form I of etoricoxib as substantially disclosed hereinbefore.

The invention is further illustrated by the following examples which are provided to be exemplary of the invention and do not limit the scope of the invention.

### Example 1:

**Table 1**

| **S. No.** | **Ingredient** | ***Quantity (% w*/*w)*** |
|---|---|---|
| 1 | Etoricoxib | 30.00 |
| 2 | Sodium lauryl Sulfate | 2.50 |
| 3 | Low substituted Hydroxy Propyl Cellulose (L-HPC LH-11) | 3.75 |
| 4 | Microcrystalline Cellulose | 30.25 |
| 5 | Calcium Hydrogen Phosphate (Anhydrous) | 28.00 |
| 6 | Croscarmellose sodium | 1.50 |
| 7 | Magnesium Stearate | 0.50 |
| 8 | Croscarmellose sodium | 1.50 |
| 9 | Colloidal Silicon Dioxide | 1.50 |
| 10 | Magnesium Stearate | 0.50 |

**Process:** Etoricoxib, sodium lauryl sulphate, low substituted hydroxyl propyl cellulose, microcrystalline cellulose, calcium hydrogen phosphate and croscarmellose sodium were mixed together and lubricated with magnesium stearate. The blend was then dry granulated using roll compactor to achieve desired granules. Blend the granules with remaining quantity of croscarmellose sodium and colloidal silicon dioxide followed by lubrication with magnesium stearate and compressed using suitable punch tooling.

### Example 2:

### (not according to the invention)

**Table 2**

| **S. No.** | **Ingredient** | ***Quantity (% w*/*w)*** |
|---|---|---|
| 1 | Etoricoxib (EDMF, CHL) (Form I) | 30.00 |
| 2 | Low substituted Hydroxy Propyl Cellulose (L-HPC LH-11) | 3.75 |
| 3 | Microcrystalline Cellulose | 32.75 |
| 4 | Calcium Hydrogen Phosphate (Anhydrous) | 12.50 |
| 5 | Croscarmellose sodium | 1.50 |
| 6 | Colloidal Silicon Dioxide (Aerosil 200) | 1.00 |
| 7 | Magnesium Stearate | 0.50 |
| 8 | Croscarmellose sodium | 1.00 |
| 9 | Calcium Hydrogen Phosphate (Anhydrous) | 15.00 |
| 10 | Colloidal Silicon Dioxide (Aerosil 200) | 1.50 |
| 11 | Magnesium Stearate | 0.50 |

**Process:** Etoricoxib, low substituted hydroxyl propyl cellulose, microcrystalline cellulose, calcium hydrogen phosphate, croscarmellose sodium and colloidal silicon dioxide were mixed together and lubricated with magnesium stearate. The blend was then dry granulated using roll compactor to achieve desired granules. Blend the granules with remaining quantity of croscarmellose sodium and colloidal silicon dioxide followed by lubrication with magnesium stearate and compressed using suitable punch tooling.

### Dissolution study of Examples 1 & 2:

Table 3 provides dissolution data of etoricoxib tablet prepared as per Example 1, 2 and reference formulation (Arocixa® Tablet). For determination of drug release rate, USP Type II (Paddle) apparatus (50rpm) was used wherein 900ml of pH 6.8 Phosphate buffer was used as medium.

**Table 3**

| **Time (min)** | **% Drug Release** | | |
|---|---|---|---|
| | Reference Product Arocixa® 120 mg | Example 1 | Example 2 |
| 15 | 48 | 65 | 46 |
| 45 | 72 | 79 | 63 |
| 90 | 85 | 85 | 74 |

## Claims

1. A pharmaceutical composition comprising crystalline Form I of etoricoxib, at least one solubility enhancing agent which is a surfactant selected from the group consisting of anionic surfactants, cationic surfactants, non-ionic surfactants and zwitterionic surfactants or mixtures thereof, and one or more pharmaceutically acceptable excipients,
wherein the anionic surfactant is selected from the group consisting of salts of aliphatic monoesters of sulfuric acid and soaps; sulfonated aromatic agents and salts thereof; alkyl naphthalene sulfonates; sulphosuccinates; sulfated polyoxyethylated alcohols; sulfated oils; dioctyl sodium sulfosuccinate; phosphatidyl choline, phosphatidyl glycerol, phosphatidyl inosine, phosphatidylserine, phosphatidic acid and their salts; glyceryl esters; and bile acids and salts thereof;
wherein the non-ionic surfactant is selected from the group consisting of polyoxyethylene fatty alcohol ethers; polyoxyethylene sorbitan fatty acid esters; polyoxyethylene fatty acid esters; sorbitan esters; glycerol monostearate; polyethylene glycols; polypropylene glycols; cetyl alcohol; cetostearyl alcohol; stearyl alcohol; aryl alkyl polyether alcohols; polyoxyethylene-polyoxypropylene copolymers; and polaxamines; and
wherein the composition is prepared by dry granulation and said etoricoxib retains its original polymorphic form.

2. A pharmaceutical composition of claim 1, wherein the cationic surfactant is selected from the group consisting of benzalkonium chloride, cetyl trimethyl ammonium bromide, dodecyl dimethyl ammonium bromide, hexadecyl (cetyl) trimethylammonium bromide, dodecyl pyridinium chloride, lauryl dimethyl benzyl ammonium chloride, acyl carnitine hydrochlorides, alkyl pyridinium halides, and dodecylamine hydrochloride.

3. A pharmaceutical composition of claim 1, wherein the zwitterionic surfactant is selected from the group consisting of cocamidopropyl betaine, sodium cocoamphoacetate, cocamidopropyl hydroxysultaine, and sodium cocamphopropionate.

4. A pharmaceutical composition of claim 1, comprising the crystalline Form I of etoricoxib having D₉₀ particle size of less than 250 µm.

5. A pharmaceutical composition of any preceding claim, wherein the amount of solubility enhancing agent ranges from 0.01% to 10% w/w of the composition.

6. A pharmaceutical composition of any preceding claim, wherein the solubility enhancing agent is sodium lauryl sulphate.

7. A process for preparing pharmaceutical composition comprising:
a. mixing crystalline Form I of etoricoxib, a solubility enhancing agent which is a surfactant selected from the group consisting of anionic surfactants, cationic surfactants, non-ionic surfactants and zwitterionic surfactants or mixtures thereof, and one or more pharmaceutical excipients;
wherein the anionic surfactant is selected from the group consisting of salts of aliphatic monoesters of sulfuric acid and soaps; sulfonated aromatic agents and salts thereof; alkyl naphthalene sulfonates; sulphosuccinates; sulfated polyoxyethylated alcohols; sulfated oils; dioctyl sodium sulfosuccinate; phosphatidyl choline, phosphatidyl glycerol, phosphatidyl inosine, phosphatidylserine, phosphatidic acid and their salts; glyceryl esters; and bile acids and salts thereof;
wherein the non-ionic surfactant is selected from the group consisting of polyoxyethylene fatty alcohol ethers; polyoxyethylene sorbitan fatty acid esters; polyoxyethylene fatty acid esters; sorbitan esters; glycerol monostearate; polyethylene glycols; polypropylene glycols; cetyl alcohol; cetostearyl alcohol; stearyl alcohol; aryl alkyl polyether alcohols; polyoxyethylene-polyoxypropylene copolymers; and polaxamines;
b. dry granulating the above mixture to achieve desired granules; and
c. lubricating the granules of step (b) with lubricant and converting the resulting mixture into a solid dosage form.

8. A process of claim 7, wherein the cationic surfactant is selected from the group consisting of benzalkonium chloride, cetyl trimethyl ammonium bromide, dodecyl dimethyl ammonium bromide, hexadecyl (cetyl) trimethylammonium bromide, dodecyl pyridinium chloride, lauryl dimethyl benzyl ammonium chloride, acyl carnitine hydrochlorides, alkyl pyridinium halides, and dodecylamine hydrochloride.

9. A process of claim 7, wherein the zwitterionic surfactant is selected from the group consisting of cocamidopropyl betaine, sodium cocoamphoacetate, cocamidopropyl hydroxysultaine, and sodium cocamphopropionate.

10. A process of any of claims 7 to 9, wherein the solid dosage form is tablet.

11. A process of any of claims 7 to 9, wherein the solid dosage form is capsule.

## Patentansprüche

1. Pharmazeutische Zusammensetzung, umfassend eine kristalline Form I von Etoricoxib, mindestens ein die Löslichkeit verbesserndes Mittel, das ein Tensid ist, ausgewählt aus der Gruppe, bestehend aus anionischen Tensiden, kationischen Tensiden, nichtionischen Tensiden und zwitterionischen Tensiden oder Gemischen davon und einem oder mehr pharmazeutisch verträglichen Hilfsstoff(en),
wobei das anionische Tensid aus der Gruppe ausgewählt ist, bestehend aus Salzen von aliphatischen Monoestern von Schwefelsäure und Seifen; sulfonierten aromatischen Mitteln und Salzen davon; Alkylnaphthalensulfonaten; Sulfosuccinaten, sulfatierten polyoxyethylierten Alkoholen; sulfatierten Ölen, Dioctylnatriumsulfosuccinat; Phosphatidylcholin, Phosphatidylglycerol, Phosphatidylinosin, Phosphatidylserin, Phosphatidsäure und ihren Salzen; Gycerylestern; und Gallensäuren und Salzen davon;
wobei das nichtionische Tensid aus der Gruppe ausgewählt ist, bestehend aus Polyoxyethylenfettalkoholethern; Polyoxyethylensorbitanfettsäureestern; Polyoxyethylenfettsäureestern; Sorbitanestern; Glycerolmonostearat; Polyethylenglycolen, Polypropylenglycolen; Cetylalkohol; Cetostearylalkohol; Stearylalkohol; Arylalkylpolyetheralkoholen; Polyoxyethylen-Polyoxypropylen-Copolymeren; und Polaxaminen; und
wobei die Zusammensetzung durch Trockengranulierung hergestellt wird und das genannte Etoricoxib seine ursprüngliche polymorphe Form beibehält.

2. Pharmazeutische Zusammensetzung nach Anspruch 1, wobei das kationische Tensid aus der Gruppe ausgewählt ist, bestehend aus Benzalkoniumchlorid, Cetyltrimethylammoniumbromid, Dodecyldimethylammoniumbromid, Hexadecyl(cetyl)-trimethylammoniumbromid, Dodecylpyridiniumchlorid, Lauryldimethylbenzylammoniumchlorid, Acylcarnitinhydrochloriden, Akylpyridiniumhalogeniden und Dodecylaminhydrochlorid.

3. Pharmazeutische Zusammensetzung nach Anspruch 1, wobei das zwitterionische Tensid aus der Gruppe ausgewählt ist, bestehend aus Cocamidopropylbetain, Natriumcocoamphoacetat, Cocamidopropylhydroxysultain und Natriumcocamphopropionat.

4. Pharmazeutische Zusammensetzung nach Anspruch 1, umfassend die kristalline Form I von Etoricoxib mit einer D₉₀-Partikelgröße von weniger als 250 µm.

5. Pharmazeutische Zusammensetzung nach einem der vorangehenden Ansprüche, wobei die Menge des die Löslichkeit verbessernden Mittels im Bereich von 0,01 Gew.-% bis 10 Gew.-% der Zusammensetzung liegt.

6. Pharmazeutische Zusammensetzung nach einem der vorangehenden Ansprüche, wobei das die Löslichkeit verbessernde Mittel Natriumlaurylsulfat ist.

7. Verfahren zum Herstellen einer pharmazeutischen Zusammensetzung, umfassend:
a. Mischen einer kristallinen Form I von Etoricoxib, eines die Löslichkeit verbessernden Mittels, das ein Tensid ist, ausgewählt aus der Gruppe, bestehend aus anionischen Tensiden, kationischen Tensiden, nichtionischen Tensiden und zwitterionischen Tensiden oder Gemischen davon und einem oder mehr pharmazeutischen Hilfsstoff(en);
wobei das anionische Tensid aus der Gruppe ausgewählt ist, bestehend aus Salzen von aliphatischen Monoestern von Schwefelsäure und Seifen; sulfonierten aromatischen Mitteln und Salzen davon; Alkylnaphthalensulfonaten; Sulfosuccinaten, sulfatierten polyoxyethylierten Alkoholen; sulfatierten Ölen, Dioctylnatriumsulfosuccinat; Phosphatidylcholin, Phosphatidylglycerol, Phosphatidylinosin, Phosphatidylserin, Phosphatidsäure und ihren Salzen; Gycerylestern; und Gallensäuren und Salzen davon;
wobei das nichtionische Tensid aus der Gruppe ausgewählt ist, bestehend aus Polyoxyethylenfettalkoholethern; Polyoxyethylensorbitanfettsäureestern; Polyoxyethylenfettsäureestern; Sorbitanestern; Glycerolmonostearat; Polyethylenglycolen, Polypropylenglycolen; Cetylalkohol; Cetostearylalkohol; Stearylalkohol; Arylalkylpolyetheralkoholen; Polyoxyethylen-Polyoxypropylen-Copolymeren; und Polaxaminen;
b. Trockengranulieren des vorstehenden Gemischs zum Erzielen des gewünschten Granulats; und
c. Gleitfähigmachen des Granulats von Schritt (b) mit einem Gleitmittel und Umwandeln des sich ergebenden Gemischs in eine feste Arzneiform.

8. Verfahren nach Anspruch 7, wobei das kationische Tensid aus der Gruppe ausgewählt ist, bestehend aus Benzalkoniumchlorid, Cetyltrimethylammoniumbromid, Dodecyldimethylammoniumbromid, Hexadecyl(cetyl)-trimethylammoniumbromid, Dodecylpyridiniumchlorid, Lauryldimethylbenzylammoniumchlorid, Acylcarnitinhydrochloriden, Alkylpyridiniumhalogeniden und Dodecylaminhydrochlorid.

9. Verfahren nach Anspruch 7, wobei das zwitterionische Tensid aus der Gruppe ausgewählt ist, bestehend aus Cocamidopropylbetain, Natriumcocoamphoacetat, Cocamidopropylhydroxysultain und Natriumcocamphopropionat.

10. Verfahren nach einem der Ansprüche 7 bis 9, wobei die feste Arzneiform eine Tablette ist.

11. Verfahren nach einem der Ansprüche 7 bis 9, wobei die feste Arzneiform eine Kapsel ist.

## Revendications

1. Composition pharmaceutique comprenant la forme cristalline I de l'étoricoxib, au moins un agent améliorant la solubilité qui est un tensioactif sélectionné dans le groupe constitué de tensioactifs anioniques, de tensioactifs cationiques, de tensioactifs non ioniques et de tensioactifs zwittérioniques ou de mélanges de ceux-ci, et un ou plusieurs excipients pharmaceutiquement acceptables,
dans laquelle le tensioactif anionique est sélectionné dans le groupe constitué de sels de monoesters aliphatiques d'acide sulfurique et de savons ; d'agents aromatiques sulfonés et de sels de ceux-ci ; de sulfonates d'alkylnaphtalène ; de sulfosuccinates ; d'alcools polyoxyéthylés sulfatés ; d'huiles sulfatées ; du sulfosuccinate de dioctyle et de sodium ; de la phosphatidylcholine, du phosphatidyl-glycérol, de la phosphatidylinosine, de la phosphatidylsérine, de l'acide phosphatidique et de sels de ceux-ci ; d'esters glycéryliques ; et d'acides biliaires et de sels de ceux-ci ;
dans laquelle le tensioactif non ionique est sélectionné dans le groupe constitué d'éthers d'alcools gras et de polyoxyéthylène ; d'esters d'acides gras et de polyoxyéthylène-sorbitane ; d'esters d'acides gras et de polyoxyéthylène ; d'esters de sorbitane ; du monostéarate de glycérol ; de polyéthylènes glycols ; de polypropylènes glycols ; de l'alcool cétylique ; de l'alcool cétostéarylique ; de l'alcool stéarylique ; de polyétheralcools arylalkyliques ; de copolymères de polyoxyéthylène-polyoxypropylène ; et de polaxamines ; et
la composition étant préparée par granulation à sec et ledit étoricoxib conservant sa forme polymorphe originale.

2. Composition pharmaceutique selon la revendication 1, dans laquelle le tensioactif cationique est sélectionné dans le groupe constitué du chlorure de benzalkonium, du bromure de cétyltriméthylammonium, du bromure de dodécyldiméthylammonium, du bromure d'hexadécyl(cétyl)trimétylammonium, du chlorure de dodécylpyridinium, du chlorure de lauryldiméthylbenzylammonium, de chlorhydrates d'acylcarnitine, d'halogénures d'alkylpyridinium, et du chlorhydrate de dodécylamine.

3. Composition pharmaceutique selon la revendication 1, dans laquelle le tensioactif zwittérionique est sélectionné dans le groupe constitué de la cocamidopropylbétaïne, du cocoamphoacétate de sodium, de la cocamidopropylhydroxysultaïne, et du cocamphopropionate de sodium.

4. Composition pharmaceutique selon la revendication 1, comprenant la forme cristalline I de l'étoricoxib ayant une taille de particule D₉₀ inférieure à 250 µm.

5. Composition pharmaceutique selon l'une quelconque des revendications précédentes, dans laquelle la quantité d'agent améliorant la solubilité est de 0,01 % à 10 % p/p de la composition.

6. Composition pharmaceutique selon l'une quelconque des revendications précédentes, dans laquelle l'agent améliorant la solubilité est le laurylsulfate de sodium.

7. Procédé de préparation d'une composition pharmaceutique, comprenant :
a. le mélange de la forme cristalline I de l'étoricoxib, d'au moins un agent améliorant la solubilité qui est un tensioactif sélectionné dans le groupe constitué de tensioactifs anioniques, de tensioactifs cationiques, de tensioactifs non ioniques et de tensioactifs zwittérioniques ou de mélanges de ceux-ci, et d'un ou plusieurs excipients pharmaceutiques ;
dans lequel le tensioactif anionique est sélectionné dans le groupe constitué de sels de monoesters aliphatiques d'acide sulfurique et de savons ; d'agents aromatiques sulfonés et de sels de ceux-ci ; de sulfonates d'alkylnaphtalène ; de sulfosuccinates ; d'alcools polyoxyéthylés sulfatés ; d'huiles sulfatées ; du sulfosuccinate de dioctyle et de sodium ; de la phosphatidylcholine, du phosphatidyl-glycérol, de la phosphatidylinosine, de la phosphatidylsérine, de l'acide phosphatidique et de sels de ceux-ci ; d'esters glycéryliques ; et d'acides biliaires et de sels de ceux-ci ;
dans lequel le tensioactif non ionique est sélectionné dans le groupe constitué d'éthers d'alcools gras et de polyoxyéthylène ; d'esters d'acides gras et de polyoxyéthylène-sorbitane ; d'esters d'acides gras et de polyoxyéthylène ; d'esters de sorbitane ; du monostéarate de glycérol ; de polyéthylènes glycols ; de polypropylènes glycols ; de l'alcool cétylique ; de l'alcool cétostéarylique ; de l'alcool stéarylique ; de polyétheralcools arylalkyliques ; de copolymères de polyoxyéthylène-polyoxypropylène ; et de polaxamines ;
b. la granulation à sec du mélange ci-dessus pour obtenir les granulés souhaités ; et
c. la lubrification des granulés de l'étape (b) avec un lubrifiant et la conversion du mélange ainsi obtenu en une forme galénique solide.

8. Procédé selon la revendication 7, dans lequel le tensioactif cationique est sélectionné dans le groupe constitué du chlorure de benzalkonium, du bromure de cétyltriméthylammonium, du bromure de dodécyldiméthylammonium, du bromure d'hexadécyl(cétyl)trimétylammonium, du chlorure de dodécylpyridinium, du chlorure de lauryldiméthylbenzylammonium, de chlorhydrates d'acylcarnitine, d'halogénures d'alkylpyridinium, et du chlorhydrate de dodécylamine.

9. Procédé selon la revendication 7, dans lequel le tensioactif zwittérionique est sélectionné dans le groupe constitué de la cocamidopropylbétaïne, du cocoamphoacétate de sodium, de la cocamidopropylhydroxysultaïne, et du cocamphopropionate de sodium.

10. Procédé selon l'une quelconque des revendications 7 à 9, dans lequel la forme galénique solide est un comprimé.

11. Procédé selon l'une quelconque des revendications 7 à 9, dans lequel la forme galénique solide est une capsule.
